# EUROPEAN PATENT SPECIFICATION

(11) **EP 2 797 682 B1**
(45) Date of publication and mention of the grant of the patent: **27.04.2016**
(21) Application number: 12812155.5
(22) Date of filing: 20.12.2012
(51) Int. Cl.: B01J 19/24, C12M 1/34, F28D 1/06, F28F 3/12, F28F 9/02, B01J 19/00, F28D 21/00

(54) **SYSTEM FOR CONTROLLING THE TEMPERATURE OF A BIOREACTOR**
SYSTEM ZUR REGELUNG DER TEMPERATUR EINES BIOREAKTORS
SYSTÈME DE COMMANDE DE LA TEMPÉRATURE D'UN BIORÉACTEUR

(30) Priority: 29.12.2011 DK 201101012; 24.09.2012 DK 201200584
(43) Date of publication of application: 05.11.2014
(73) Proprietor: Service-teknikker.dk ApS, 3000 Helsingør (DK)
(72) Inventor: BUCH, Anders, DK-3000 Helsingør (DK)
(74) Representative: Olesen, Birthe Bjerregaard
(86) International application number: PCT/DK2012/050492
(87) International publication number: WO 2013/097861

(56) References cited:
- DE-A1-102007 040 487
- GB-A- 1 125 341
- US-A- 4 213 498
- US-A1- 2008 063 771

## Description

### Field of the Invention

The present invention relates to a system for controlling the temperature of small scaled bioreactors or soft media bags.

### Background of the Invention

When using fermenters and bioreactor in the pharmaceutical and biotech branches - use is normally made of different equipment for controlling the optimal temperature during the processes taking place in such fermenters or bioreactor.

And for such purposes it is known to made use of expensive fermenters or bioreactors comprising exterior walls having a complicated built-in tubular temperature controlling system, through which a fluid heating or cooling medium is circulated by means of exterior heating or cooling systems belonging to the equipment of the laboratory in question.

An example of a cooling or heating jackets for mounting around a container, e.g. a bioreactor, is shown in US 4213498 A. The flexible heat exchanger comprises a belt of plastic, in particular polyethylene (PE) or polypropylene (PP), having a number of parallel internal ducts for heat exchanging media. The belt is connected to manifolds at each end.

GB 1125341 A discloses another heating or cooling jacket. The belt is made of flexible metallic sheets and the internal flow channel is made by embossing the channel in the sheets of flexible metal.

US 2008/0063771 A discloses another flexible heating or cooling jacket, which consists of a flexible belt having open flow channels arranged into one side of the belt. The flexible belt is attached in watertight manner to the container by arranging the open flow channels on the exterior surface of the container.

DE 10 2007 040 487 A1 describes a heating or cooling jacket consisting of a flexible belt with tube connections as inlets /outlets for the heating /cooling fluid. The heating or cooling jacket is suitable for single use in sterile environment or may be sterilisable.

### Object of the Invention

The present invention has for its purpose to provide a new and improved system for controlling the temperature of the introductory mentioned type and which by means of simple provisions makes it possible to achieve a very significant and reliable temperature controlling system for use in connection with fermenters or bioreactors of different sizes in particular in the form of bottle-shaped fermenters or bioreactors or soft media bags, and especially fermenters/bioreactors or similar containers of the single-use type.

### Description of the Invention

The temperature controlling system said tubular channel system being arranged as mutually parallel tubular channels provided plate shaped body member and adapted to surround said bioreactor and to be in close contact with the exterior wall of said bioreactor, and that said tubular channel system being adapted to be supplied with heating or cooling medium from an exterior temperature controlling system by means of manifolds attached to each ends of the plate shaped body member.

By means of simple provisions it will hereby be possible to achieve a very significant and reliable system for controlling the temperature of fermenters or bioreactors of different sizes in particular in the form of simple bottle-shaped fermenters or bioreactors of the single-use type.

The system according to invention is special in that said plate-shaped body member is made from silicone rubber.

Hereby is furthermore achieved that said bendable mantle or jacket together with the used bioreactor may be autoclave proof - if not made use of a sub or the like. Sub is an abbreviation for single-use bioreactor. In addition, the likelihood of tearing the parts apart is even further reduced since silicone rubber has a high tensile strength. Additionally, silicone rubber can withstand autoclavation.

Similarly, the bendable mantle or jacket together may be made autoclave proof when the manifold is made of a heat resistant material such as stainless steel, aluminium, or a heat resistant resin such as polytetrafluor ethylene (PTFE), also known as Teflon or a homo- or copolymer of polyoxymethylene (POM). POM is able to withstand the temperatures in an autoclave, e.g. 140 °C, in shorter periods and will thus be very suitable material as an alternative to Teflon, especially in case the bendable mantles or jackets are used as sterilized single use equipment, e.g. under sterile conditions in the pharmaceutical or biotech industry or laboratories.

In yet another embodiment, the system according to the invention may furthermore be such provided that opposite ends of said bendable mantle or jacket are adapted to be connected with manifold or connection means for establishing fluid proof connections with said tubular channel system By using manifolds comprises a housing having a receiving part for insertion of the plate-shaped body member and in which a number of protrusions or ribs are arranged at the interior wall of the manifold for engaging with the exterior sides of said plate-shaped body member and an separate interior connector comprising a number of tube shaped stubs. The number of tube shaped stubs are optionally connected by a connector plate thus forming a separate interior connector. This establishes a firm connection by the tube shaped stubs engaging with the interior sides the tubular channels of the plate-shaped body member in order to fasten the ends of the plate-shaped body member in the manifolds, since the tube shaped stubs squeeze the walls of the plate shaped body against the interior surface of the manifolds.

Alternatively, said manifold or connection means is with a thickness smaller than that of said plate-shaped body member, and that said manifold or connection means at opposite sides comprises plate-shaped connection means, which at an interior side having a projecting sharp edge for engaging with the exterior sides of said plate-shaped body member, and where the width between the plate-shaped connection means is smaller than the thickness of said plate-shaped body member.

The embodiments having the mechanical fastening means between the manifold and the plate-shaped body described above are more reliable than prior art connection means, e.g. glueing the parts together, they possess large tensile strength, i.e. the parts are not easily torn apart. The former of the mechanical fastening means according to the invention posses an even larger tensile strength in when compared to the latter. In addition, in case the manifold is made of Teflon (PTFE) it is not possible to glue the manifold to the plate-shaped body member. The connection between the plate-shaped body and the manifold according to the present invention needs no glueing and thus solves the problem of attaching a Teflon manifold to the plate-shaped body.

In yet another embodiment, the system according to the invention may advantageously be such provided that the length of said plate-shaped body member is adapted to be easily adjusted in order together with said manifold or connection means to accommodate the circumference of any bioreactor.

And correspondingly the system according to the invention may furthermore be such provided that a number of said plate-shaped body members are interconnected next to each other, i.e. in parallel, by means of a number of interconnected manifold or connection means or by attaching a number of plate-shaped body members in parallel between the manifolds to accommodate the height of any bioreactor.

I another embodiment a stack of at least two of the said plate-shaped body members are interconnected on top of each other by means of at least two stacked and interconnected manifolds or by attaching a number of plate-shaped body members in stacked manner between the manifolds. This provides an even further improved temperature control when used in connection with small scale fermenters. In addition this embodiment enables the temperature controlling system according to the present invention to be used in controlling the temperature in liquids contained in soft media bags, e.g. infusion bags. The soft media bags are simply inserted into the openings between two adjacent plate-shaped body members in a stack. This further enables that temperature is efficiently controlled in the media contained in the bag, e.g. when the soft media bag and the surrounding temperature controlling system according to the present invention is placed on a reciprocating plate or the like for mixing of the content in the media bag.

In order to make the mounting easy the system according to the invention may be such provided that said bendable mantle or jacket is adapted to be held in close contact to the outside of said bioreactor by means of surrounding hoop-like quick closure means, or by means of strips, straps, rubber bands or the like attached to projections on the manifolds.

Furthermore, in order to achieve the best possible mode of operation and flexibility the present invention also relates to an alternative system for controlling the temperature in a single-wall bioreactor - or controlling the temperature in any further single-wall bottle type containers for supplying any fluid additives to the bioreactor - and of the type comprising a tubular channel system through which a fluid heating or cooling medium is adapted to be circulated, which system is characterised in that said tubular channel system being arranged in a bendable mantle or jacket adapted to surround said bioreactor or any supply bottle and to be in close contact with the exterior wall of said bioreactor or any supply bottle, and that said tubular channel system being adapted to be supplied with heating or cooling fluid from an exterior temperature controlling system.

The above described system for controlling the temperature in small scale bioreactors is thus likewise also particularly suitable for maintaining a constant temperature in other small scale containers such as supply containers, e.g. bottles etc. in which it is important to maintain the content at a constant temperature.

### Description of the Drawing

In the following the invention will be described in more details with reference to the drawing, in which:
- Fig. 1: shows a perspective, sectional view of an embodiment for a half-part of a bendable mantle or jacket according to the invention,
- Fig. 2: shows a perspective view of an embodiment for a bendable mantle or jacket comprising manifold or connection means according to the invention,
- Fig. 3: shows a perspective view of an embodiment similar to that of Fig. 2 - but shown with both manifold or connection means mounted,
- Fig. 4: shows a perspective split view illustrating the assembling of the different parts of an embodiment for an extra high bendable mantle or jacket according to the invention,
- Fig. 5: shows a perspective view of an extra high bendable mantle or jacket made from the parts shown in Fig. 4,
- Fig. 6: shows a perspective view of the bendable mantle or jacket shown in Fig. 5, but seen from another angle,
- Fig. 7: shows a perspective view of a bottle-shaped bioreactor mounted with an embodiment for a bendable mantle or jacket according to the invention,
- Fig. 8: shows a perspective view of a higher bottle-shaped bioreactor mounted with a bendable mantle or jacket assembled from two interconnected mantles or jackets according to the invention,
- Fig. 9: shows a perspective view of another bioreactor with built-in impeller mounted with a bendable mantle or jacket according to the invention,
- Fig. 10: shows a plane view of an alternative embodiment for a bendable mantle or jacket according to the invention,
- Fig. 11: shows a perspective view of the bendable mantle or jacket from Fig. 9 mounted around the outside of an alternative bioreactor,
- Fig. 12: shows a perspective split view of an embodiment of the bendable mantle or jacket in a single mantle version,
- Fig. 13: shows a perspective view of the assembled bendable mantle or jacket in the single mantle version,
- Fig. 14: shows a perspective split view of another embodiment of the bendable mantle or jacket in a double mantle version,
- Fig. 15: shows a crossview of a manifold used in an alternative embodiment of the bendable mantle or jacket,
- Fig. 16: shows a perspective view of an embodiment of the bendable mantle or jacket in a version having two layers of the plate-shaped body member, and
- Fig. 17: show a cross view of a manifold in the dual layered embodiment shown in fig. 16.

### Detailed Description of the Invention

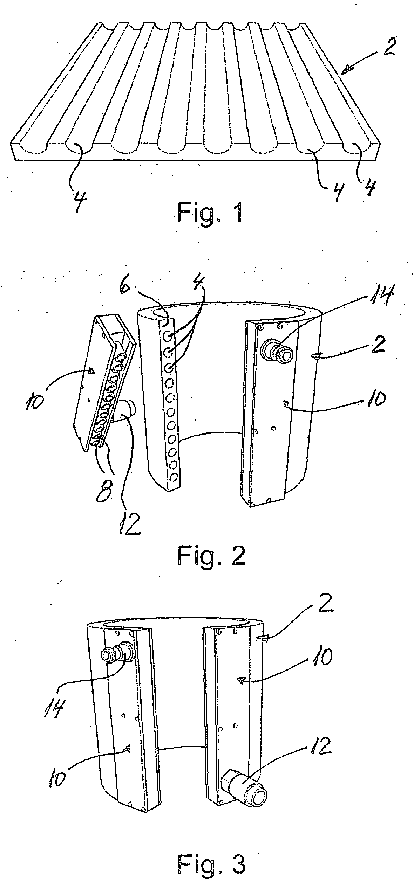

Fig. 1 shows a perspective, sectional view of a half-part of a preferred embodiment for a plate-shaped body member 2 made by moulding preferably from silicone rubber. Said body member 2 is provided with a number of mutually parallel arranged tubular channels 4.

Figs. 2 and 3 show that opposite ends 6 of said tubular channels 4 are adapted to be fluid-proof connected with tubular connecting stubs 8 of manifold or connection means 10. The latter is provided with either an inlet stub 12 or an outlet stub 14.

Preferable both stubs 12 and 14 are parts of so-called quick couplings for instant connection with complementary coupling parts of tubes belonging to an exterior heating or cooling system.

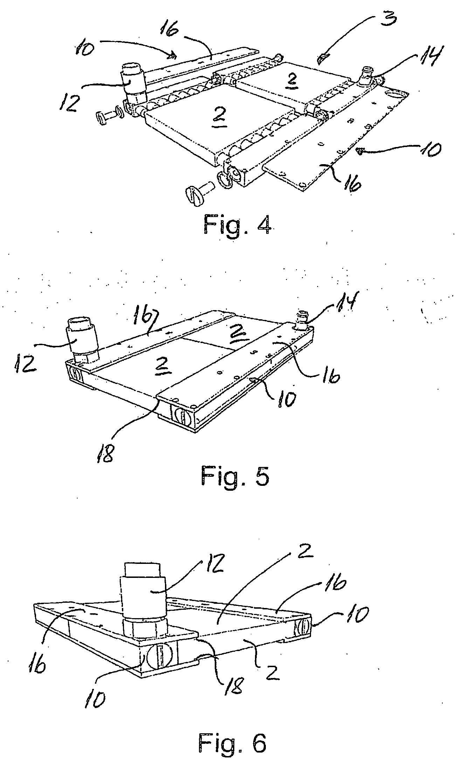

Fig. 4 illustrates how the different parts of the bendable body member 2 of the mantle or jacket 3 are assembled to form the complete bendable mantle or jacket 3 shown in Figs. 5 and 6. The tubular connection stubs 8 of the manifold or connection means 10 are inserted into opposite open ends of the tubular channels 4.

Afterwards these fluid-proof connections are secured by means of outer connection plates 16, which by means of screws are mounted at opposite side of the not-compressible manifold or connection means 10 so that projecting sharp edges 18 along the inner sides of said connection plates 16 are forced into opposite surfaces of the relative soft bendable body member 2.

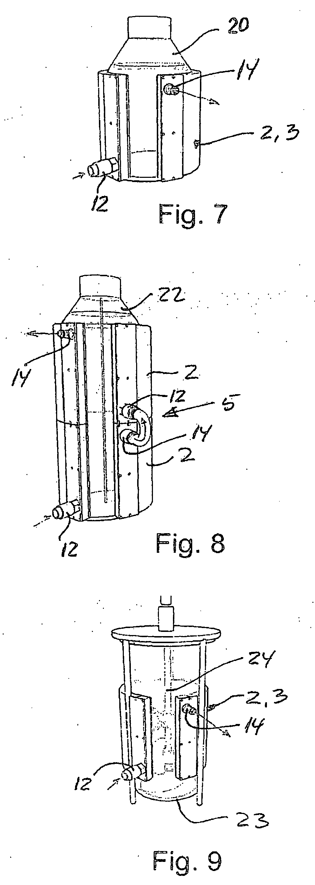

Fig. 7 shows how a complete bendable mantle or jacket 3 is mounted around a bioreactor 20 so that said inlet stub 12 is positioned at the bottom of the mantle or jacket 3 while the outlet stub 14 is positioned at the upper side of said mantle or jacket 3.

Fig. 8 shows how a complete bendable mantle or jacket 5 comprising two bendable mantle or jackets 3 are mounted next to each other, i.e. in parallel around a taller bioreactor 22, and how the respective inlet stubs 12 and outlet stubs 14 are interconnected to insure the correct flow direction of fluid from said exterior heating or cooling system from the bottom to the top of the respective bendable mantle or jackets 3 and 5.

Fig. 9 shows how a complete bendable mantle or jacket 3 is mounted around another type of bioreactor 23 comprising a built-in rotating impeller 24.

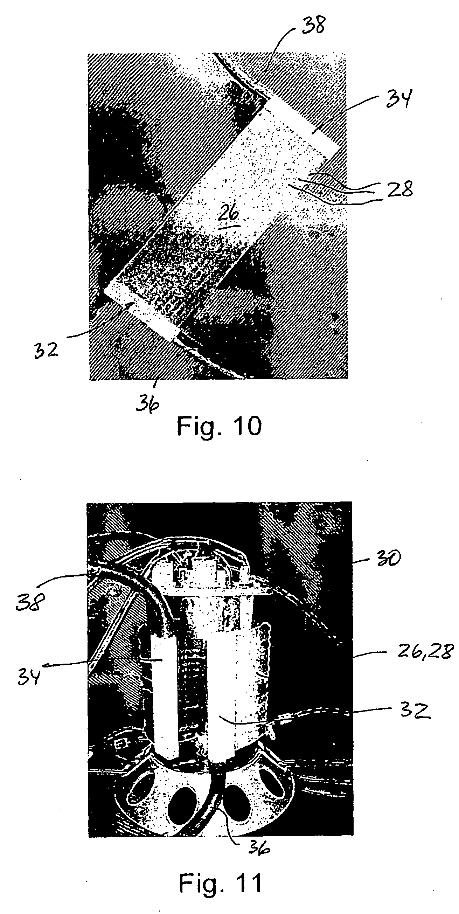

Fig. 10 shows an alternative embodiment for a bendable mantle or jacket 26 according to the invention - where the mantle or jacket 26 consists of a number of mutually arranged and sidewardly interconnected bendable tubes 28 with a length accommodating the circumference of a third bioreactor 30 (Fig. 11).

The opposite ends of said tubes 28 are fluid-proof connected to an inlet manifold 32 respective an outlet manifold 34, which by means of tube connections 36 and 38 are connected to an exterior heating or cooling system.

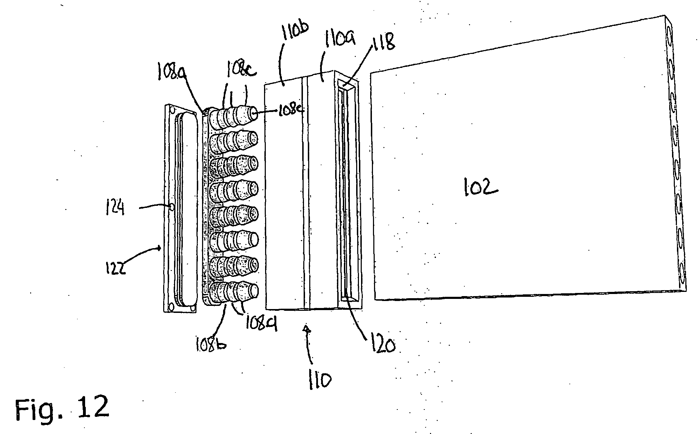

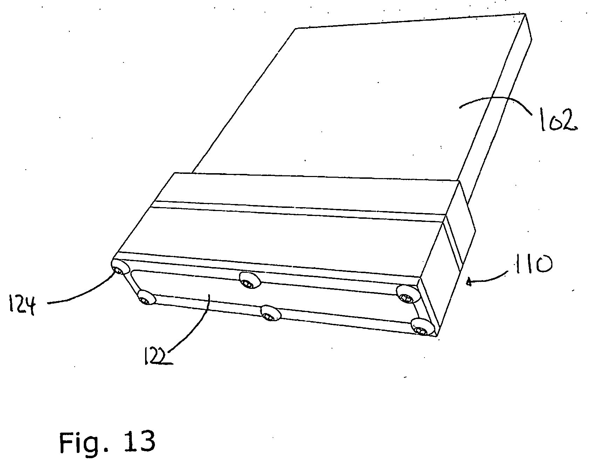

Figs. 12-15 show an alternative connection between the manifolds 110 and the plate-shaped body member 2, 102. Inlet 12 and outlet 12 (not shown in figs. 12- 15) stubs for heating/coiling media are arranged at the manifold as described above. Similarly, the plate-shaped body member 102 is essentially identical to the plate-shaped body member 2 described above.

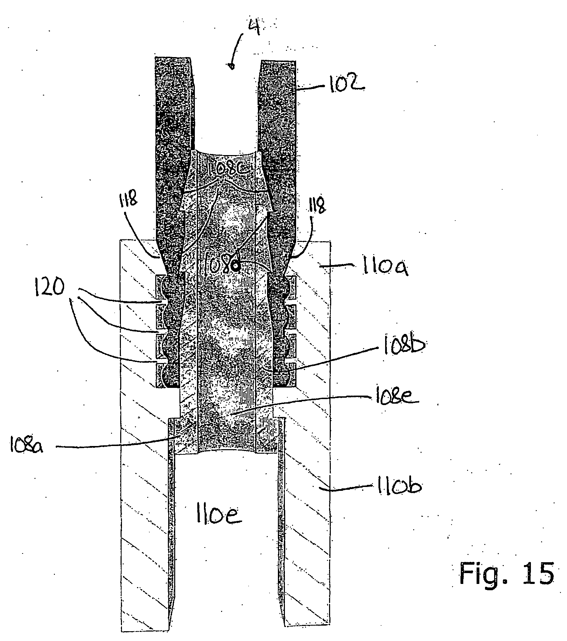

The manifold 110 comprises a connector part 110a which is intended to be connected to the plate-shaped body member 102, and a manifold inlet/outlet part 110b which is connected to the inlet stub 12 /outlet stub 14, which are separated by a partition wall or a projecting rib 110c, as can be seen in fig. 15.

A number of parallel extending ribs 120 are arranged at the internal wall of the manifold connector part 110a, preferably in parallel to the partition wall/projecting rib 110c, and whose function will be described below.

An interior connector 108 is intended to be mounted inside the manifold 110a as described below. The internal connector comprises a connector plate 108a onto which a number of tube shaped stubs 108b are mounted. Each tubular stub 108a has an outer wall having a number of conically shaped parts 108c having an edge 108d. The connector plate 108a comprises a number of through-going holes corresponding to the fluid channel 108e inside the tubular stubs 108b in order to establish fluid connection between interior fluid chamber 110e in the manifold inlet/outlet part and the parallel tubular channels 4 in the flexible plate shaped body member 102. Alternatively, the connection is established by a number of individual tubular stubs 108b, i.e. without the connector plate 108a, which are mounted separately into each opening in the tubular channels 4 in the flexible plate shaped body member 102.

The partition wall 110c in the manifold 110 comprises a large opening or a row of smaller, preferably circular openings 110d which allow the tubular stubs 108b to enter into the manifold connector part 110a when inserted from the manifold inlet/outlet part 110b. Thus, the bendable mantle or jacket is assembled by inserting the flexible plate shaped body member 102 into the manifold connector part 110a. Thereafter, the internal connector 108 is inserted by passing the tubular stubs 108b through the opening/openings 110d and into the ends of the parallel tubular channels 4 in the flexible plate shaped body member 102. Hereby fluid connection between the manifold inlet/outlet chamber 110e and the parallel tubular channels in the flexible plate-shaped body member 102 is established.

This alternative connection establishes a firm connection between the plate-shaped body member 102 and the manifolds 110, by squeezing the ends of the plate-shaped body member 102 between the manifold wall internal ribs 120 and the outer surface of the tubular stubs 108b on the interior connector 108. This connection has an increased tensile strength, i.e. the parts are not easily torn apart, and it is therefore less likely that the bendable mantle or jacket is torn apart when forces are pulling in the manifold parts 110, since the ends of the flexible plate-shaped body member 102 is squeezed firmly between the ribs 120 and the internal connector 108.

Finally, the end plate 122 is attached to the manifold housing 110, e.g. by glueing, screwing or similar conventional attachment means 124 as indicated on figs. 12, 13 and 14.

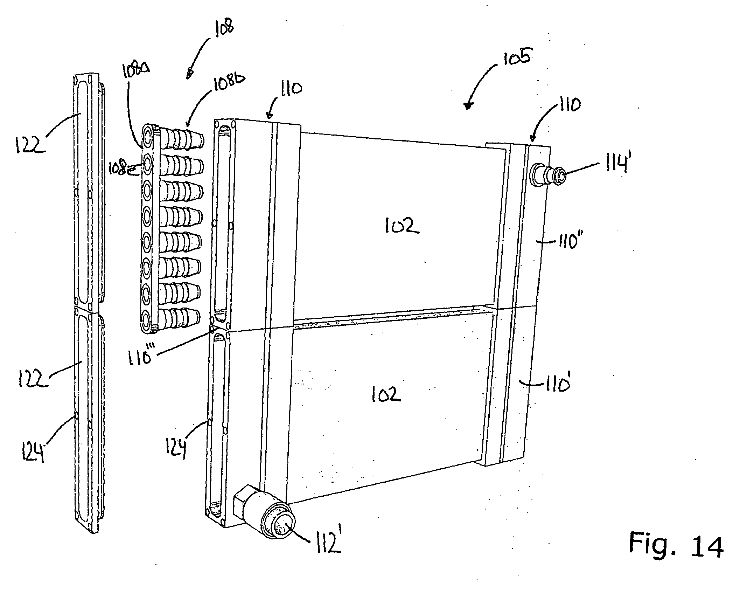

Fig. 14 shows an alternative version of the bendable mantle or jacket. This embodiment is assembled in a similar way as described above. The manifolds 110 shown in fig. 14 are intended to be coupled to two parallel bendable plate- shaped body members 102, as illustrated in fig. 15. The manifold parts 110 may be made in one piece, or alternatively two standard manifolds 110' and 110" are connected in parallel as shown, e.g. by screwing, glueing or the like well known methods, except that that glueing is not used for Teflon (PTFE) manifolds. In case this "twin version" 110',110" of the manifold 110 is used, fluid connection is established between the manifold half parts by drilling a passage (not shown) through the wall 110"'

When the plate-shaped body member 102 is made of silicone rubber, the likelihood of tearing the parts apart is even further reduced since silicone rubber has a higher tensile strength than other resins, which might otherwise be suitable for making the bendable plate-shaped body member 2, 102. Additionally, silicone rubber can withstand autoclavation.

Thus the bendable mantle or jacket according to the invention has a significantly increased lifetime when compared to similar prior art heating/cooling jackets.

In a particularly suitable embodiment of the bendable mantle or jacket, the manifold 10, 110 is made of teflon (PTFE) and the plate-shaped body member 2, 102 is made of silicone rubber.

In Fig. 11 the bendable mantle or jacket 26 is temporarily held in place around the bioreactor 30 during the photo session by means of transparent tape - but of course more proper and reliable fixation of the mantle or jacket 26 should be arranged for, e.g. by providing a number of projections on each of the inlet and outlet manifolds. These projections may be used to attach one or more rubber bands, strips, straps or the like in order in order to hold the flexible heat exchanger firmly in position on the bioreactor. Alternatively, the attachment means comprise a hoop-like quick closure.

The most important purpose of the present invention is to provide a system as claimed which by means of rather simple provisions makes it possible control the temperature of any bioreactor with a significant accuracy - by way of example in a temperature level of about 37° with a temperature difference of only +/- 0.1 ° C.

In addition, the bendable mantle or jacket is made of materials which can be sterilized in an autoclave, which is important or even essential, when the system is used in connection with bioreactors, which are used for growing cell cultures under sterile conditions.

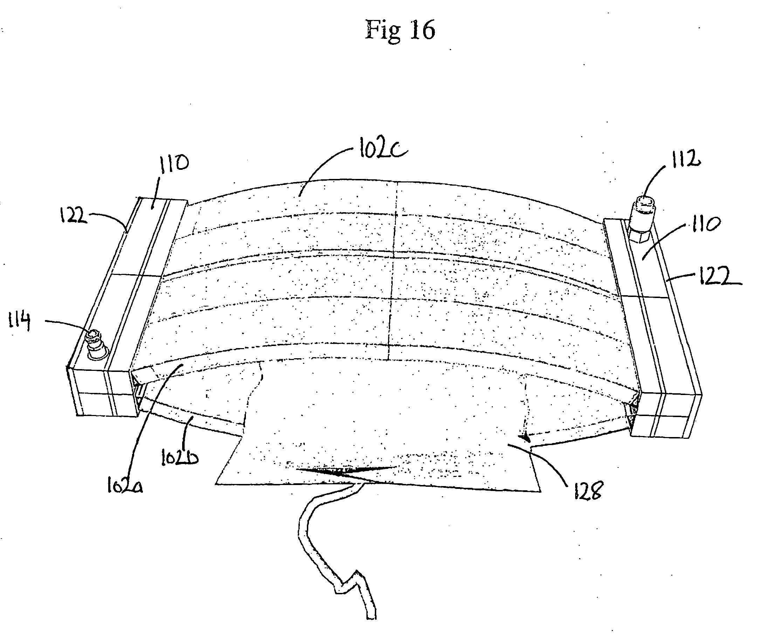

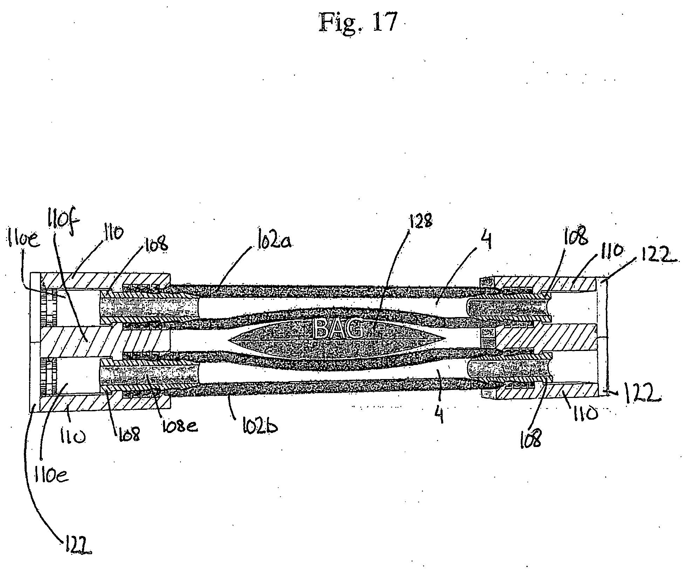

An alternative version of the bendable mantle or jacket is shown I fig. 16-17. In this embodiment at least two plate-shaped, bendable body members 102a, 102b are stacked on top of each other. This embodiment is assembled in a similar way as described above and as shown in fig. 12-15. The manifolds 110 shown in fig. 16-17 are intended to be coupled to at least two stacked bendable plate- shaped body members 102, as illustrated in the cross view of fig. 17. The manifold parts 110 may be made in one piece, or alternatively two standard manifolds 110' and 110" are mounted in stack-wise manner and connected as discussed above and fluid connection is established through the wall 11 Of between the manifold parts, e.g. by drilling a passage (not shown) through the wall 110f.

Apart from being used as described above, in order to further improve temperature control in single walled bioreactors, this embodiment is also particularly suitable for controlling the temperature in liquids contained in soft media bags 128, e.g. infusion bags. A soft media bag 128, e.g. an infusion bag, is arranged in the opening between two stacked plate-shaped bodies 102a, 102b and heating/cooling media is applied to the manifolds as discussed above. This enables keeping the liquid in the soft media bag at constant temperature as effective as discussed above for rigid bottle shaped bioreactors 20, 22. I addition, the heating/cooling jacket according to this embodiment is particularly useful when keeping the media bag and its content at constant temperature when the media bag is placed on a reciprocating plate or the like for mixing of the content in the media bag.

In principle three or more two plate-shaped, bendable body members 102 are stacked on top of each other, thus enabling two or more soft media bags to be inserted in each of the openings between the stacked plate-shaped body members 102a,102b.

As shown in fig. 16 two or more stacks 102c of plate-shaped body members 102a, 102b may be mounted in parallel in a manner similar to the embodiment shown in and as discussed in connection with figs. 4-6 or 14, thus providing a larger heat exchanging area to each soft media bag 128.

These embodiment will also be useful in connection with keeping infusion liquids at a constant temperature, e.g. during surgery, because the temperature control equipment according to the present invention can be used for soft media bags, e.g. infusion bags, and because it can withstand being sterilized in an autoclave.

The size of simple bottle-shaped (single-wall) single-use bioreactor may within the system according to the invention vary from 5 ml - 20 litres preferably from 10 ml - 10 litres or 1 litre - 10 litres. The size of the soft media bags may within the system according to the invention vary from 5 ml - 5 litres preferably from 10 ml - 2 litres or 0.2 litres - 1.5 litres, and is typically 0.5 - 1.0 litres.

The described plate-shaped, bendable body member 2, 102 of silicone rubber will be available in supply rolls in lengths of meters from which any lengths may be cut to accommodate the circumference of any possible bioreactor.

The width of said plate-shaped, bendable body member 2, 102 consisting of silicone rubber will preferably be about 80 mm - with a number of 8 mutually parallel tubular channels.

The thickness of said plate-shaped, bendable body member 2, 102 will preferable be about 10 mm and the inner diameter of the tubular channels is preferably about 6 mm.

Finally it should be mentioned that said plate-shaped body member may be provided in any other suitable manner within the scope of the invention - or said in other words the important main scope of the present invention is by simple provisions to arrange for a flexible system for controlling the temperature of primary cheap bottle-shaped (single-wall) single-use bioreactor, where a relative soft plate-shaped body member as claimed may be used in connection with any existing exterior temperature controlling system belonging to usual laboratory equipment using a fluid medium.

Reference numbers used in the drawing:
- 2: plate-shaped body member
- 3: single mantle or jacket
- 4: tubular channels
- 5: double mantle or jacket
- 6: opposite end of tubular channels
- 8: tubular connection stubs
- 10: manifold or connection means
- 12: inlet stub
- 14: outlet stub
- 16: outer connection plates
- 18: sharp projection edges
- 20: single bioreactor
- 22: double bioreactor
- 23: another type of bioreactor
- 24: a built-in impeller
- 26: mantle or jacket
- 28: bendable tubes
- 30: a third bioreactor
- 32: inlet manifold
- 34: outlet manifold
- 36: connection tube
- 38: connection tube
- 102: plate-shaped body member
- 103: single mantle or jacket
- 104: tubular channels
- 105: double mantle or jacket
- 108: interior connector with tubular connection stubs
- 110: manifold or connection means
- 112: inlet stub
- 114: outlet stub
- 116: end plates
- 118: opening for inserting plate shaped body member 102
- 120: ribs
- 122: end plates
- 124: end plate mounting holes
- 126: end plate mounting means
- 128: soft media bag or infusion bag

## Claims

1. A small scale bioreactor or similar container (20, 22) temperature controlling system, and of the type comprising a tubular channel system (4) through which a fluid heating or cooling medium is adapted to be circulated, said tubular channel system (4) being arranged as mutually parallel tubular channels (4) provided in a plate-shaped body member (2, 102) and adapted to surround said bioreactor (20, 22) and to be in close contact with the exterior wall of said bioreactor (20, 22), and that said tubular channel system (4) being adapted to be supplied with heating or cooling medium from an exterior temperature controlling system by means of manifolds (10, 110) attached to each ends of the plate-shaped body member (2, 102), **characterised in that** said plate-shaped body member (2, 102) is made from silicone rubber.

2. A system according to claim 1, **characterised in that** that said manifold (110) comprises a housing having a receiving part (110b) for insertion of the plate-shaped body member (2, 102) and in which a number of protrusions or ribs (120) are arranged at the interior wall of the manifold (110) for engaging with the exterior sides of said plate-shaped body member (2, 102) and an interior connector (108) comprising a number of tube shaped stubs (108b), for engaging with the interior sides the tubular channels (4) of the plate-shaped body member (2, 102) in order to fasten the ends of the plate-shaped body member (2, 102) in the manifolds (10, 110) and wherein the tube shaped stubs 108b are optionally mounted on a connector plate (108a).

3. A system according to any of the claims 1-2, **characterised in that** that said manifold (10) at opposite sides comprises plate-shaped connection means (16), which at an interior side having a projecting sharp edge (18) for engaging with the exterior sides of said plate-shaped body member (2) and where the width between the plate-shaped connection means (16) is smaller than the thickness of said plate-shaped body member (2).

4. A system according to any of the claims 1-3, **characterised in that** said manifold (10, 110) is made from not-compressible material such as stainless steel, aluminium, a heat resistant resin, such as polytetraflourethylene (PTFE) or a polyoxymethylene homo- or copolymer (POM).

5. A system according to any of the claims 1-4, **characterised in that** the length of said plate-shaped body member (2, 102) is adapted to be easily adjusted in order together with said manifold (10, 110) to accommodate the circumference of any bioreactor (20, 22).

6. A system according to any of the claims 1-5, **characterised in that** a number of said plate-shaped body members (2, 102) may be interconnected next each other by means of a number of parallel and interconnected manifolds (10, 110) or by attaching a number of plate-shaped body members (2, 102) in parallel between the manifolds (10, 110) to accommodate the height of any bioreactor (20, 22).

7. A system according to any of the claims 1-6, **characterised in that** at least two of the said plate-shaped body members (2, 102) may be interconnected on top of each other by means of at least two stacked and interconnected manifolds (10, 110) or by attaching a number of plate-shaped body members (2, 102) in parallel between the manifolds (10, 110).

8. A system according to any of the preceding claims, **characterised in that** said bendable mantle or jacket (3, 5) is adapted to be held in close contact to the outside of said bioreactor (20, 22) by means of surrounding hoop-like quick closure means or by means of strips, straps, rubber bands or the like attached to projections on the manifolds (10, 110).

9. A system for controlling the temperature in a single-wall bioreactor or controlling the temperature in any single-wall bottle type containers for supplying any fluid additives to a bioreactor - and of the type comprising a tubular channel system (4) through which a fluid heating or cooling medium is adapted to be circulated, **characterised in that** said tubular channel system (4) being arranged in a bendable mantle or jacket (3, 5) adapted to surround said bioreactor or any supply bottle and to be in close contact with the exterior wall of said bioreactor or any supply bottle, and that said tubular channel system (4) being adapted to be supplied with heating or cooling fluid from an exterior temperature controlling system, and wherein said tubular channel system (4) is arranged in a plate-shaped body member (2, 102) made from silicone rubber.

## Patentansprüche

1. Temperaturregelsystem für einen kleinen Bioreaktor oder ähnlichen Behälter (20, 22) des Typs, der ein rohrförmiges Kanalsystem (4) umfasst, wobei ein Fluidheiz- oder -kühlmedium eingerichtet ist, um dadurch hindurch zirkuliert zu werden, wobei das rohrförmige Kanalsystem (4) als gegenseitig parallele rohrförmige Kanäle (4) angeordnet ist, die in einem plattenförmigen Körperelement (2, 102) bereitgestellt und eingerichtet sind, um den Bioreaktor (20, 22) zu umgeben und in engem Kontakt mit der Außenwand des Bioreaktors (20, 22) zu stehen, und wobei das rohrförmige Kanalsystem (4) eingerichtet ist, um mit dem Heiz- oder Kühlmedium von einem Außentemperaturregelsystem mittels Verteilern (10, 110) versorgt zu werden, die mit jedem Ende des plattenförmigen Körperelements (2, 102) verbunden sind, **dadurch gekennzeichnet, dass** das plattenförmige Körperelement (2, 102) aus Silikonkautschuk besteht.

2. System nach Anspruch 1, **dadurch gekennzeichnet, dass** der Verteiler (110) ein Gehäuse umfasst, das einen Aufnahmeteil (110b) zum Einführen des plattenförmigen Körperelements (2, 102) aufweist, wobei eine Anzahl von Vorsprüngen oder Rippen (120) an der Innenwand des Verteilers (110) angeordnet ist, um mit den Außenseiten des plattenförmigen Körperelements (2, 102) in Eingriff zu gehen, und einen inneren Verbinder (108), der eine Anzahl rohrförmiger Stummel (108b) umfasst, um mit den Innenseiten der rohrförmigen Kanäle (4) des plattenförmigen Körperelements (2, 102) in Eingriff zu gehen, um die Enden des plattenförmigen Körperelements (2, 102) in den Verteilern (10, 110) zu befestigen, und wobei die rohrförmigen Stummel (108b) optional auf einer Verbinderplatte (108a) montiert sind.

3. System nach einem der Ansprüche 1-2, **dadurch gekennzeichnet, dass** der Verteiler (10) an entgegengesetzten Seiten plattenförmige Verbindungseinrichtungen (16) aufweist, die auf einer Innenseite eine hervorstehende scharfe Kante (18) aufweisen, um mit den Außenseiten des plattenförmigen Körperelements (2) in Eingriff zu gehen, und wobei die Breite zwischen den plattenförmigen Verbindungseinrichtungen (16) kleiner als die Dicke des plattenförmigen Körperelements (2) ist.

4. System nach einem der Ansprüche 1-3, **dadurch gekennzeichnet dass** der Verteiler (10, 110) aus nicht komprimierbarem Material wie Edelstahl, Aluminium, einem hitzebeständigen Harz, z. B. Polytetraflourethylen (PTFE) oder einem Polyoxymethylenhomo- oder -copolymer (POM) besteht.

5. System nach einem der Ansprüche 1 bis 4, **dadurch gekennzeichnet, dass** die Länge des plattenförmigen Körperelements (2, 102) eingerichtet ist, um leicht zusammen mit dem Verteiler (10, 110) eingestellt zu werden, um den Umfang von jedem Bioreaktor (20, 22) aufzunehmen.

6. System nach einem der Ansprüche 1 bis 5, **dadurch gekennzeichnet, dass** eine Anzahl der plattenförmigen Körperelemente (2, 102) nebeneinander mittels einer Anzahl paralleler und miteinander verbundener Verteiler (10, 110) oder durch Befestigen einer Anzahl plattenförmiger Körperelemente (2, 102) parallel zwischen den Verteilern (10, 110) miteinander verbunden sein können, um die Höhe von jedem Bioreaktor (20, 22) aufzunehmen.

7. System nach einem der Ansprüche 1 bis 6, **dadurch gekennzeichnet, dass** mindestens zwei der plattenförmigen Körperelemente (2, 102) aufeinander mittels mindestens zweier gestapelter und miteinander verbundener Verteiler (10, 110) oder durch Befestigen einer Anzahl plattenförmiger Körperelemente (2, 102) parallel zwischen den Verteilern (10, 110) miteinander verbunden sein können.

8. System nach einem der vorangegangenen Ansprüche, **dadurch gekennzeichnet, dass** der biegbare Mantel oder die Hülle (3, 5) eingerichtet ist, um mittels umgebender ringförmiger Verschlusseinrichtungen oder mittels Einrichtungen von Streifen, Riemen, Gummibändern oder dergleichen, die an Vorsprüngen auf den Verteilern (10, 110) befestigt sind, in engem Kontakt zu der Außenseite des Bioreaktors (20, 22) gehalten zu werden.

9. System zum Regeln der Temperatur in einem einwandigen Bioreaktor oder Regeln der Temperatur in jedem beliebigen einwandigen flaschenartigen Behälter zur Versorgung eines Bioreaktors mit Fluidzusätzen, und des Typs, der ein rohrförmiges Kanalsystem (4) umfasst, wobei ein Fluidheiz- oder -kühlmedium eingerichtet ist, um dadurch hindurch zirkuliert zu werden, **dadurch gekennzeichnet, dass** das rohrförmige Kanalsystem (4) in einem biegbaren Mantel oder einer Hülle (3, 5) angeordnet ist, der/die eingerichtet ist, um den Bioreaktor oder jede beliebige Versorgungsflasche zu umgeben, und in engem Kontakt zu der Außenwand des Bioreaktors oder jeder Versorgungsflasche zu stehen, und dass das rohrförmige Kanalsystem (4) eingerichtet ist, dass ihm Heiz- oder Kühlfluid von einem Außentemperaturregelsystem zugeführt wird, und wobei das rohrförmige Kanalsystem (4) in einem plattenförmigen Körperelement (2, 102) angeordnet ist, das aus Silikonkautschuk besteht.

## Revendications

1. Système de commande de la température d'un bioréacteur ou récipient similaire (20, 22) à petite échelle et du type comprenant un système de canaux tubulaires (4) à travers lequel un milieu chauffant ou refroidissant fluide est adapté pour circuler, ledit système de canaux tubulaires (4) étant disposé sous forme de canaux tubulaires (4) mutuellement parallèles fournis dans un élément de corps en forme de plaque (2, 102) et adaptés pour entourer ledit bioréacteur (20, 22) et être en contact étroit avec la paroi extérieure dudit bioréacteur (20, 22), et ledit système de canaux tubulaires (4) étant adapté pour être alimenté en milieu chauffant ou refroidissant provenant d'un système de commande de la température extérieur au moyen de collecteurs (10, 110) fixés à chaque extrémité de l'élément de corps en forme de plaque (2, 102), **caractérisé en ce que** ledit élément de corps en forme de plaque (2, 102) est réalisé en caoutchouc silicone.

2. Système selon la revendication 1, **caractérisé en ce que** ledit collecteur (110) comprend un boîtier ayant une partie de réception (110b) pour l'insertion de l'élément de corps en forme de plaque (2, 102) et dans lequel un nombre de protubérances ou nervures (120) est disposé au niveau de la paroi intérieure du collecteur (110) pour la mise en prise avec les faces extérieures dudit élément de corps en forme de plaque (2, 102) et un connecteur intérieur (108) comprenant un nombre d'embouts en forme de tube (108b), pour la mise en prise avec les faces intérieures des canaux tubulaires (4) de l'élément de corps en forme de plaque (2, 102) afin de fixer les extrémités de l'élément de corps en forme de plaque (2, 102) dans les collecteurs (10, 110) et dans lequel les embouts en forme de tube (108b) sont montés optionnellement sur une plaque de connecteur (108a).

3. Système selon l'une quelconque des revendications 1-2, **caractérisé en ce que** ce dit collecteur (10) au niveau de faces opposées comprend des moyens de raccordement en forme de plaque (16), ayant au niveau d'une face intérieure une arête vive en saillie (18) pour la mise en prise avec les faces extérieures dudit élément de corps en forme de plaque (2) et où la largeur entre les moyens de raccordement en forme de plaque (16) est inférieure à l'épaisseur dudit élément de corps en forme de plaque (2).

4. Système selon l'une quelconque des revendications 1-3, **caractérisé en ce que** ledit collecteur (10, 110) est réalisé en matériau non compressible comme l'acier inoxydable, l'aluminium, une résine résistante à la chaleur, comme du polytétrafluoroéthylène (PTFE) ou un homopolymère ou copolymère polyoxyméthylène (POM).

5. Système selon l'une quelconque des revendications 1-4, **caractérisé en ce que** la longueur dudit élément de corps en forme de plaque (2, 102) est adaptée pour s'ajuster facilement afin d'accommoder conjointement avec ledit collecteur (10, 110) la circonférence de tout bioréacteur (20, 22).

6. Système selon l'une quelconque des revendications 1-5, **caractérisé en ce qu'**un nombre desdits éléments de corps en forme de plaque (2, 102) peut être raccordé l'un à côté de l'autre au moyen d'un nombre de collecteurs (10, 110) raccordés et parallèles ou par fixation d'un nombre d'éléments de corps en forme de plaque (2, 102) en parallèle entre les collecteurs (10, 110) pour accommoder la hauteur de tout bioréacteur (20, 22).

7. Système selon l'une quelconque des revendications 1-6, **caractérisé en ce qu'**au moins deux desdits éléments de corps en forme de plaque (2, 102) peuvent être raccordés l'un sur l'autre au moyen d'au moins deux collecteurs (10, 110) raccordés et empilés ou par fixation d'un nombre d'éléments de corps en forme de plaque (2, 102) en parallèle entre les collecteurs (10, 110).

8. Système selon l'une quelconque des revendications précédentes, **caractérisé en ce que** ladite enveloppe ou chemise pliable (3, 5) est adaptée pour être maintenue en contact étroit avec l'extérieur dudit bioréacteur (20, 22) au moyen de moyens à fermeture rapide de type cerceau encerclant ou au moyen de bandes, sangles, bracelets en caoutchouc ou similaires fixés aux saillies sur les collecteurs (10, 110).

9. Système de commande de la température d'un bioréacteur à une seule paroi ou de commande de la température de tout récipient de type bouteille à une seule paroi pour amener tout additif fluide à un bioréacteur - et du type comprenant un système de canaux tubulaires (4) à travers lequel un milieu chauffant ou refroidissant fluide est adapté pour circuler, **caractérisé en ce que** ledit système de canaux tubulaires (4) étant disposé dans une enveloppe ou chemise pliable (3, 5) adaptée pour entourer ledit bioréacteur ou toute bouteille d'alimentation et être en contact étroit avec la paroi extérieure dudit bioréacteur ou de toute bouteille d'alimentation, et ce dit système de canaux tubulaires (4) étant adapté pour être alimenté en fluide chauffant ou refroidissant provenant d'un système de commande de la température extérieur et dans lequel ledit système de canaux tubulaires (4) est disposé dans un élément de corps en forme de plaque (2, 102) en caoutchouc silicone.
